(19) Europäisches Patentamt
European Patent Office
Office européen des brevets

(11) **EP 0 814 781 B1**

(12) **EUROPÄISCHE PATENTSCHRIFT**

(45) Veröffentlichungstag und Bekanntmachung des Hinweises auf die Patenterteilung:
**13.02.2002 Patentblatt 2002/07**

(21) Anmeldenummer: **96907412.9**

(22) Anmeldetag: **09.03.1996**

(51) Int Cl.$^7$: **A61K 9/20**

(86) Internationale Anmeldenummer:
**PCT/EP96/01019**

(87) Internationale Veröffentlichungsnummer:
**WO 96/29060 (26.09.1996 Gazette 1996/43)**

(54) **LAGERSTABILE ARZNEIFORMEN**

LONG-SHELF-LIFE MEDICAMENTS

FORMES GALENIQUES STABLES AU STOCKAGE

(84) Benannte Vertragsstaaten:
**AT BE CH DE DK ES FI FR GB IE IT LI NL PT SE**

(30) Priorität: **21.03.1995 DE 19509806**

(43) Veröffentlichungstag der Anmeldung:
**07.01.1998 Patentblatt 1998/02**

(73) Patentinhaber: **BASF AKTIENGESELLSCHAFT 67056 Ludwigshafen (DE)**

(72) Erfinder:
- **BREITENBACH, Jörg**
  **D-68199 Mannheim (DE)**
- **RIEGER, Jens**
  **D-67069 Ludwigshafen (DE)**
- **ROSENBERG, Joerg**
  **D-67158 Ellerstadt (DE)**
- **SANNER, Axel**
  **D-67227 Frankenthal (DE)**

(56) Entgegenhaltungen:
**EP-A- 0 481 968**

**Beschreibung**

Die vorliegende Erfindung betrifft feste Arzneiformen, erhältlich durch Extrusion einer lösungsmittelfreien Schmelze, enthaltend neben einem oder mehreren Wirkstoffen eine Mischung aus

> a) 10 bis 99 Gew.-% eines oder mehrerer wasserlöslicher, thermoplastisch verarbeitbarer Homo- oder Copolymere des N-Vinylpyrrolidons,

> b) 1 bis 90 Gew.-% abgebaute Stärken, und

> c) 0 bis 50 Gew.-% eines oder mehrerer üblicher Pharmahilfsstoffe,

wobei die Mengenangaben sich auf die Summe der Mengen a), b) und gegebenenfalls c) beziehen,
und anschließender Formgebung zu kugel- oder linsenförmigen Teilchen mit Durchmessern von 0,5 bis 4 mm oder durch Hindurchführen des extrudierten Stranges zwischen zwei gegenläufig angetriebene Walzen mit einander gegenüberliegenden Vertiefungen im Walzenteil, wobei die Form der Vertiefungen die Tablettenform bestimmt.

Die Herstellung fester Arzneiformen durch Extrusion einer Schmelze, enthaltend neben dem Wirkstoff Polymerisate auf Basis von N-Vinylpyrrolidon, mit anschließender Formgebung ist beispielsweise aus der EP-B 240 904 bekannt.

In der WO 93/10758 werden Retard-Arzneiformen auf Basis einer amorphen Kohlenhydratglasmatrix beschrieben, die als Mittel zur Rekristallisation Polyvinylpyrrolidon oder Maltodextrine enthalten. Zur Herstellung der Matrix werden wäßrige Lösungen der Matrixkomponenten solange erhitzt bis viskose Mischungen erhalten werden, in die der Wirkstoff durch Kneten eingearbeitet wird, worauf die wirkstoffhaltige Mischung beispielsweise durch Extrusion aufgearbeitet werden kann.

In der EP-A 0 481 968 sind Arzneiformen offenbart, welche durch Extrusion einer lösungsmittelfreien Schmelze, enthaltend beispielsweise einen Wirkstoff, ein Polymer wie Polyvinylpyrrolidone, Maltodextrin sowie weitere übliche Hilfsstoffe, wobei die Arzneiformen als abreißbare, perforierte Bänder erhalten werden.

Bei der Herstellung fester Arzneiformen durch Extrusion wirkstoffhaltiger Schmelzen besteht eine grundsätzliche Schwierigkeit darin, daß die zur Matrixbildung verwendeten Polymere einerseits eine ausreichende thermoplastische Verarbeitbarkeit aufweisen müssen, andererseits aber in der fertigen Arzneiform auch bei längerer Lagerung formstabil bleiben. Eine gute thermoplastische Verarbeitbarkeit ist aber vor allem bei solchen Polymeren gegeben, die entweder relativ niedrige Molekulargewichte und damit relativ niedrige Glasübergangstemperaturen aufweisen und/oder weichmachende Monomere wie beispielsweise Vinylacetat enthalten, d.h. gerade diese Polymeren erzeugen bei Verarbeitung zu festen Arzneiformen das unerwünschte Phänomen des "kalten Flusses".

Aufgabe der vorliegenden Erfindung war es, lagerstabile feste Arzneiformen zu finden, die auf einfache Weise durch Extrusion wirkstoffhaltiger Schmelzen und anschließender Formgebung erhältlich sind.

Demgemäß wurden die eingangs definierte Arzneiformen gefunden.

Geeignete Wirkstoffe sind beispielsweise:

Betamethason, Thioctsäure, Sotalol, Salbutamol, Norfenefrin, Silymarin, Dihydroergotamin, Buflomedil, Etofibrat, Indometacin, Oxazepam, beta-Acetyldigoxim, Piroxicam, Haloperidol, ISMN, Amitriptylin, Diclofenac, Nifedipin, Verapamil, Pyritinol, Nitrendipin, Doxycyclin, Bromhexin, Methylprednisolon, Clonidin, Fenofibrat, Allopurinol, Pirenzepin, Levothyroxin, Tamoxifen, Metildigoxin, o-(beta-Hydroxyethyl)rutosid, Propicillin, Aciclovirmononitrat, Paracetamol, Naftidrofuryl, Pentoxyfyllin, Propafenon, Acebutolol, L-Thyroxin, Tramadol, Bromocriptin, Loperamid, Ketotifen, Fenoterol, Ca-Dobelisat, Propanolol, Minocyclin, Nicergolin, Ambroxol, Metoprolol, beta-Sitosterin, Enalaprilhydrogenmaleat, Bezafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibenclamid, Cefaclor, Etilefrin, Cimetidin, Theophyllin, Hydromorphon, Ibuprofen, Primidon, Clobazam, Oxaceprol, Medroxyprogesteron, Flecainid, Mg-Pyridoxal-5-phosphatglutaminat, Hymechromon, Etofyllinclofibrat, Vincamin, Cinnarizin, Diazepam, Ketoprofen, Flupentixol, Molsidomin, Glibornurid, Dimetinden, Melperon, Soquinolol, Dihydrocodein, Clomethiazol, Clemastin, Glisoxepid, Kallidinogenase, Oxyfedrin, Baclofen, Carboxymethylcystein, Thioridacin, Betahistin, L-Tryptophan, Myrtol, Bromelaine, Prenylamin, Salazosulfa pyridin, Astemizol, Sulpirid; Benserazid, Dibenzepin, Acetylsalicylsäure, Miconazol, Nystatin, Ketoconazol, Na-Picosulfat, Colestyramin, Gemfibrocil, Rifampicin, Fluorcortolon, Mexiletin, Amoxicillin, Terfenadin, Mucopolysaccharidpolyschwefelsäureester, Triazolam, Mianserin, Tiaprofensäure, Amezimiummetilsulfat, Mefloquin, Probucol, Chinidin, Carbamazepin, Mg-L-aspartat, Penbutolol, Piretanid, Amitriptylin, Cyproteron, Na-Valproinat, Mebeverin, Bisacodyl, 5-AminoSalicylsäure, Dihydralazin, Magaldrat, Phenprocoumon, Amantadin, Naproxen, Carteolol, Famotidin, Methyldopa, Auranofin, Estriol, Nadolol, Levomepromazin, Doxorubicin, Metofenazat, Azathioprin, Flutamid, Norfloxacin, Fendilin, Prajmaliumbitartrat, Aescin. Acetaminophen (= Paracetamol), Acetohexamid, Acetyldigoxim, Acetylsalicylsäure, Acromycin, Anipamil, Benzocain, beta-Carotin, Chloramphenicol, Chlordiazepoxid, Chlormadinoacetat, Chlorthiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethazid, Ibuprofen, Indimethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol (= Sulfameter), Sulfaperin, Sulfathiazol, Sulfisoxazol, Testosteron, Tolazamid, Tolbutamid, Trimethoprim, Tyrothricin.

Auch Vitamine lassen sich erfindungsgemäß formulieren. Dazu gehören die Vitamine der A-Gruppe, der B-Gruppe, wobei neben B1, B2, B6 und B12 sowie Nicotinsäure und Nicotinamid auch Verbindungen mit Vitamin 3-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und $\alpha$-Liponsäure. Weiterhin Vitamine der C-Gruppe, D-Gruppe, E-Gruppe, F-Gruppe, H-Gruppe, I- und J-Gruppe, K-Gruppe und P-Gruppe.

Ganz besonders bevorzugte Wirkstoffe sind erfindungsgemäß Ibuprofen, Acetylsalicylsäure, Paracetamol, Phenazon, Flurbiprofen, Captopril, Nifedipin, Acetylcystein, Naftidrofuryl, Verapamil und Furosemid.

Es können auch Kombinationen von Wirkstoffen verwendet werden.

Die Wirkstoffmenge, bezogen auf die feste Arzneiform, kann je nach Wirksamkeit und Freisetzungsgeschwindigkeit in weiten Grenzen variiert werden. So kann der Wirkstoffanteil im Bereich von 0,1 bis 90, vorzugsweise 0,5 bis 60 Gew.-% liegen.

Neben den Wirkstoffen enthalten die erfindungsgemäßen Arzneiformen als matrixbildende Substanzen,

   a) 10 - 99, vorzugsweise 40 - 95 Gew.-% eines oder mehrerer wasserlöslicher thermoplastisch verarbeitbaren Homo- oder Copolymere des N-Vinylpyrrolidons,

   b) 1 - 90, vorzugsweise 5 - 60 Gew.-% einer abgebauten Stärke, und

   c) 0 bis 50 Gew.-% eines oder mehrerer üblicher pharmazeutischer Hilfsstoffe,

wobei die Mengenangaben sich auf die Summe der Mengen a), b) und gegebenenfalls c) beziehen.

Als Komponenten a) kommen wasserlösliche thermoplastisch verarbeitbare Homo- oder Copolymere des N-Vinylpyrrolidons oder Gemische solcher Polymeren in Betracht. Die Polymeren weisen üblicherweise Glasübergangstemperaturen im Bereich von 80 bis 190, bevorzugt 90 bis 175°C auf. Geeignete Homopolymere sind beispielsweise Polymere mit K-Werten nach Fikentscher im Bereich von 10 bis 30. Geeignete Copolymere können als Comonomere ungesättigte Carbonsäuren, z.B. Methacrylsäure, Crotonsäure, Maleinsäure, Itaconsäure, sowie deren Ester mit Alkoholen mit 1 bis 12, vorzugsweise 1 bis 8 Kohlenstoffatomen, ferner Hydroxyethyl- oder Hydroxypropylacrylat und -methacrylat, (Meth)acrylamid, die Anhydride und Halbester der Maleinsäure und Itaconsäure (wobei der Halbester vorzugsweise erst nach der Polymerisation gebildet wird), N-Vinylcaprolactam und Vinylpropionat, enthalten.

Bevorzugte Comonomere sind Acrylsäure und, besonders bevorzugt, Vinylacetat. Die Comonomere können in Mengen von 20 bis zu 70 Gew.-% enthalten sein. Die Herstellung der polymeren Komponenten a) ist allgemein bekannt.

Als Komponenten b) werden erfindungsgemäß wasserlösliche abgebaute Stärken (Dextrine) verwendet.

Solche Dextrine sind handelsüblich und in einfacher Weise aus Stärke durch unvollständige Hydrolyse mit verdünnter Säure, durch Hitzeeinwirkung sowie durch oxidativen oder enzymatischen Abbau mit Hilfe von Amylasen zugänglich.

Durch Hydrolyse in wäßriger Phase erhältliche Stärkeabbauprodukte eines gewichtsmittleren Molekulargewichtes von 2500 bis 25000 werden im Unterschied zu den Röstdextrinen üblicherweise als verzuckerte Stärken bezeichnet und sind als solche im Handel erhältlich.

Derartige verzuckerte Stärken sind von den Röstdextrinen u.a. dadurch chemisch verschieden, daß bei einem hydrolytischen Abbau in wäßrigem Medium (üblicherweise Suspensionen oder Lösungen), der in der Regel bei Feststoffgehalten von 10 bis 30 Gew.-% sowie vorzugsweise säure- oder enzymkatalysiert vorgenommen wird, die Möglichkeit der Rekombination und Verzweigung im wesentlichen nicht gegeben ist, was sich nicht zuletzt auch in anderen Molekulargewichtsverteilungen äußert.

Die Herstellung verzuckerter Stärken ist allgemein bekannt und u.a. in Günther Tegge, Stärke und Stärkederivate, Behr's Verlag, Hamburg 1984, S. 173 und S. 220 ff sowie in der EP-A 441 197 beschrieben. Vorzugsweise handelt es sich bei den erfindungsgemäß zu verwendenden verzuckerten Stärken um solche, deren gewichtsmittleres Molekulargewicht $M_w$ im Bereich von 4000 bis 16000, besonders bevorzugt im Bereich von 6500 bis 13000 liegt.

Die Stärkeabbauprodukte weisen Dextroseäquivalente (DE) von 2 bis 40, vorzugsweise 2 bis 30 auf. Der DE-Wert charakterisiert das Reduktionsvermögen bezogen auf das Reduktionsvermögen von wasserfreier Dextrose und wird nach DIN 10 308, Ausgabe 5.71, des Deutschen Normenausschusses Lebensmittel und landwirtschaftliche Produkte, bestimmt (vgl. auch Günther Tegge, Stärke und Stärkederivate, Behr's Verlag, Hamburg 1984, S. 305).

Besonders bevorzugte Komponenten b) sind die Maltodextrine, die DE-Werte im Bereich von 3 bis 20 aufweisen und Stärkeabbauprodukte mit Kettenlängen von 4 bis 10 Anhydroglucose-Einheiten mit einem hohen Anteil an Maltose darstellen.

Als Komponenten c) eignen sich übliche Pharmahilfsmittel wie beispielsweise Füllstoffe, Schmiermittel, Formentrennmittel, Stabilisatoren, Farbstoffe, Streckmittel oder Fließmittel sowie deren Mischungen.

Das Mischen der Wirkstoffe mit den matrixbildenden Komponenten a) und b) und gegebenenfalls den Komponenten c) kann vor oder nach dem Schmelzen der Polymeren erfolgen. Bevorzugt wird das Mischen im Extruder, vorzugsweise einen Zweischneckenextruder oder einem Einschneckenextruder mit Mischabteil.

Die Herstellung der wirkstoffhaltigen Schmelzen erfolgt im Extruder bei Temperaturen von 50 bis 180, vorzugsweise 60 bis 150°C. Der extrudierte, noch plastische Strang wird anschließend einer kontinuierlichen Verformung unterworfen, beispielsweise gemäß dem in der EP-A 240 906 beschriebenen Verfahren durch Hindurchführen des Stranges zwischen zwei gegenläufig angetriebenen Walzen mit einander gegenüberliegenden Vertiefungen im Walzenanteil, wobei die Form der Vertiefungen die Tablettenform bestimmt. Auch eine Formgebung durch Kaltabschlag kommt in Betracht.

Die Schmelzen sind lösungsmittelfrei, d.h. die Einsatzstoffe werden der Verarbeitung nicht in Form von Lösungen zugeführt und es werden weder Wasser noch organische Lösungsmittel zusätzlich zugesetzt.

Bevorzugt wird der sogenannte Heißabschlag. Dabei werden die Stränge unmittelbar nach dem Austritt aus der Düsenanordnung am Extruder durch beispielsweise rotierende Messer oder eine andere geeignete Anordnung zerkleinert, zweckmäßig in Stücke, deren Länge etwa gleich dem Strangdurchmesser ist. Diese abgeschlagenen Schmelzteilchen kühlen im Luft- oder Gasstrom so weit ab, daß die Oberfläche vor einer Berührung mit anderen Teilchen oder einer Gefäßwand bereits klebfrei ist, andererseits die Teilchen aber noch so plastisch sind daß sie durch Zusammenstöße, z.B. mit der Wandung eines angeschlossenen Cyclons, eine shärische Form erhalten. Man erhält so in einfacher Weise weitgehend kugel- oder linsenförmige Teilchen mit Durchmessern von 0.5 bis 4, vorzugsweise 0.8 bis 2 mm. Die bevorzugten kleineren Teilchen sind in erster Linie zum Füllen von Kapseln geeignet.

Gewünschtenfalls können die Arzneiformen noch mit üblichen Überzügen zur Verbesserung des Aussehens oder des Geschmacks versehen werden.

Überraschenderweise wird durch den Zusatz von abgebauten Stärken die Glasübergangstemperatur der Polymer/Stärke-Mischung synergistisch angehoben. Dies führt dazu, daß auch bei Verwendung von relativ niedermolekularen Polymeren mit entsprechend niedrigen Glasübergangstemperaturen das Phänomen des kalten Flusses verhindert wird. Zudem fördern die abgebauten Stärken auch die schnelle Freisetzung des Wirkstoffs. Es war auch nicht zu erwarten, daß die abgebauten Stärken, die als solche nicht lösungsmittelfrei extrudiert werden können und in wäßriger Lösung keine gemeinsame Phase mit den Polymeren bilden, eine so gute Verträglichkeit mit den lösungsmittelfreien Polymerschmelzen aufweisen und zu homogenen Mischungen führen würden.

Beispiele

Die Komponenten wurden in den unter den jeweiligen Beispielen angegebenen Mengenverhältnissen vorgemischt und in den Einzug eines Doppelschnecken-Extruders (Werner & Pfleiderer, ZSK 30) eingetragen. Die Schmelzextrusion erfolgte mit einem Produktdurchsatz von 3 bis 4 kg/h. Die Temperaturen der einzelnen Temperaturzonen ("Schüsse") des Extruders sowie die Temperatur der beheizten Düsenleiste ist bei den Versuchen jeweils angegeben. Die Düsenleiste wies 7 Bohrungen ä 1 mm Durchmesser auf. Die über die beheizte Extruderdüsenleiste austretenden Schmelzstränge wurden durch luftgekühlten Heißabschlag mit einem Messerwalzengranulator (4 Messer, 400-850 U/min) pelletiert.

Die Wirkstofffreisetzung wurde mittels der Rührflügelmethode (Paddle Methode nach USP XXI, US-Arzneibuch) gemessen. Diese in-vitro-Prüfungsmethode dient zur Bestimmung der Lösungsrate von wirkstoffhaltigen Formlingen, z.B. Tabletten.

Hierzu wurden 900 ml eines Phosphatpuffers mit einem pH-Wert von 6.8 mit einem Zusatz von 0.1 % Natriumlaurylsulfat in einem 1 L-Gefäß mit Rundboden auf 37 °C temperiert. Eine geeignete Menge an Pellets (ca. 300 mg) der Korngröße 1.25 bis 1.60 mm wurde eingewogen. Die Wirkstofffreisetzung der Pellets wurde in diesem No-Change-Test nach USP XXI bei einer Paddle-Drehzahl von 100 Upm nach jeweils 10 Min. Stunden UV-spektroskopisch bestimmt.

Als Stärkeabbauprodukt wurde ein handelsübliches Maltodextrin (CPUR 1910, Firma Cerestar Deutschland GmbH) mit einem $M_w$ von 10540-12640 und einem DE-Wert von 11-14 eingesetzt.

Das in den Beispielen 2 bis 4 eingesetzte Copolymer aus 60 Gew.-% Vinylpyrrolidon und 40 Gew.-% Vinylacetat wies einen K-Wert von 30 auf.

Beispiel 1

Temperaturen der Extruder-Zonen (Schüsse 1-5) 20, 80, 140, 130, 130°C, Temperatur Extruder-Kopf 130 °C, Temperatur Düsenleiste 130°C

| Wirkstoff | Komponente a | Komponente b |
|---|---|---|
| Furosemid 20 Gew.-% | Polyvinylpyrrolidon K-Wert 17 70 Gew.-% | Maltodextrin 10 Gew.-% |
| Freisetzung nach 30 Min. 100% | | |

Beispiel 2

Temperaturen der Extruder-Zonen (Schüsse 1-5) 60, 120, 120, 110, 120 °C, Temperatur Extruder-Kopf 130 °C, Temperatur Düsenleiste 120 °C

| Wirkstoff | Komponente a | Komponente b |
|---|---|---|
| Nifedipin 20 Gew.% | Vinylpyrrolidon-Vinylacetat-Copolymer 75 Gew.-% | Maltodextrin 5 Gew.-% |
| Freisetzung nach 30 Min. 80%. | | |

Beispiel 3

Temperaturen der Extruder-Zonen (Schüsse 1-5) 60, 120, 120, 120, 130 °C, Temperatur Extruder-Kopf 130 °C, Temperatur Düsenleiste 160 °C

| Wirkstoff | Komponente a | Komponente b |
|---|---|---|
| Ibuprofen 40 Gew.-% | Vinylpyrrolidon-Vinylaceta-Copolymer 50 Gew.-% | Maltodextrin 10 Gew.% |
| Freisetzung nach 30 Min. 80%. | | |

Beispiel 4

Temperaturen der Extruder-Zonen (Schüsse 1-5) 70, 130, 130, 140, 130 °C, Temperatur Extruder-Kopf 130 °C, Temperatur Düsenleiste 160 °C

| Wirkstoff | Komponente a | Komponente b |
|---|---|---|
| Captopril 20 Gew.% | Vinylpyrrolidon-Vinylacetat-Copolymer 65 Gew.-% | Maltodextrin 15 Gew.-% |
| Freisetzung nach 30 Min. 80%. | | |

Beispiel 5

Es wurden die Glasübergangstemperaturen eines Polyvinylpyrrolidon mit K-Wert 17 und des Maltodextrins sowie von Mischungen der beiden Komponenten gemessen (siehe Tabelle).

Gemessen wurden die Glastemperaturen mit einem DSC Gerät 912 + Thermal Analyzer 990 der Fa. TA Instruments. Die Temperatur- und Enthalpiekalibrierung erfolgte in üblicher Weise. Die Probeneinwaage betrug typischerweise 13 mg. Die Proben wurden zunächst mit 20K/min auf 190°C aufgeheizt und dann schnell abgekühlt. Im anschließenden zweiten aufheizenden Lauf wurde die Glastemperatur (Temperatur halber Stufenhöhe der Glasstufe in der Wärmeflußkurve) ermittelt. Dieses Meßprogramm ermöglicht durch das Einprägen einer einheitlichen thermischen Vorgeschichte einen sinnvollen Vergleich zwischen den verschiedenen Proben. Zur Bestimmung der Glastemperatur ist es im vorliegenden Fall wesentlich, das angegebene Temperaturprogramm einzuhalten. Eine Messung nach DIN 53 765 liefert irreführende Werte für die Glastemperatur, da die Proben während des Meßvorgangs thermisch geschädigt werden. Die Glastemperaturen der Mischungen wurden außerdem mit Hilfe der für Polymermischungen verwendeten Gordon-Taylor Gleichung berechnet:

$$Tg = (w1Tg1+kw2Tg2)/(w1+kw2)$$

[M. Gordon, J.S. Taylor, Journal of Applied Chemistry USSR 2 (1952) 493; zitiert nach: H.A. Schneider, Polymer 30 (1989) 771]. w1 und Tg1 sind der Gewichtsanteil bzw. die Glastemperatur der Komponente i. k ergibt sich als Fitparameter zu 0,37. Die Differenz zwischen Meßwert und gefittetem Wert liegt im Bereich des Meßfehlers (ca. 1K).

Dabei stellte sich heraus, daß die jeweils gemessenen Tg-Werte höher waren als die berechneten Tg-Werte. Daraus ergibt sich, daß durch die Mischung der Komponenten a) und b) eine synergistische Anhebung der Tg erzielt werden kann (Ergebnisse siehe Tabelle).

Tabelle

|  | PVP K17 | Maltodextrin-Anteil | | | |
|---|---|---|---|---|---|
|  | 100 % | 25 % | 50 % | 75 % | 100 % |
| Tg gemessen | 123°C | 153°C | 179°C | 182°C | 188°C |
| Tg berechnet | - | 138°C | 156°C | 172°C | - |

Beispiel 6

Versuch zur Lagerstabilität:

Es wurde eine Formulierung nach den in Beispiel 3 angegebene Bedingungen hergestellt.

| Wirstoff | Komponente a | Komponente b |
|---|---|---|
| Ibuprofen 20 Gew.-% | Polyvinylpyrrolidon K-Wert 17 76 Gew.-% | Maltodextrin 4 Gew.-% |
| Freisetzung nach 30 Min. 96,8 %. | | |

Eine Formulierung mit der Zusammensetzung

| Wirkstoff Ibuprofen 20 Gew.-% | Komponente a Polyvinylpyrrolidon K-Wert 17 80 Gew.-% |
|---|---|
| zeigte nach 30 Min. 95 % Freisetzung. | |

Beide Formulierungen wurden aus der Schmelze zu Bolus-Formen verarbeitet und die Freisetzung nach USP XXI bestimmt.

Die Formen wurden bei 50°C konstanter Temperatur im geschlossenen Gefäß für 4 Wochen eingelagert.

Anschließend zeigte nur die Formulierung mit Maltodextrin keine Veränderungen in ihrer Beschaffenheit, während die Formulierung ohne Maltodextrin stark klebte, zum Teil verlaufen war oder die Bolus-Formen deformiert waren.

**Patentansprüche**

1. Feste Arzneiformen, erhältlich durch Extrusion einer lösungsmittelfreien Schmelze, enthaltend neben einem oder mehreren Wirkstoffen eine Mischung aus

    a) 10 bis 99 Gew.-% eines oder mehrerer wasserlöslicher, thermoplastisch verarbeitbarer Homo- oder Copolymere des N-Vinylpyrrolidons,

    b) 1 bis 90 Gew.-% abgebaute Stärken, und

c) 0 bis 50 Gew.-% eines oder mehrerer üblicher Pharmahilfsstoffe,

wobei die Mengenangaben sich auf die Summe der Mengen a), b) und gegebenenfalls c) beziehen, und anschließender Formgebung zu kugel- oder linsenförmigen Teilchen mit Durchmessern von 0,5 bis 4 mm oder durch Hindurchführen des extrudierten Stranges zwischen zwei gegenläufig angetriebene Walzen mit einander gegenüberliegenden Vertiefungen im Walzenteil, wobei die Form der Vertiefungen die Tablettenform bestimmt.

**2.** Arzneiformen nach Anspruch 1, enthaltend als Komponenten b) Maltodextrine.

**3.** Arzneiformen nach Anspruch 1 oder 2, mit einem Wirkstoffanteil von 0,1 bis 90 Gew.-%.

**Claims**

**1.** A solid drug form obtainable by extrusion of a solvent-free melt comprising, besides one or more active substances, a mixture of

a) 10 - 99% by weight of one or more water-soluble, melt-processable, homo- or copolymers of N-vinylpyrrolidone,

b) 1 - 90% by weight of degraded starches, and

c) 0 - 50% by weight of one or more conventional pharmaceutical ancillary substances,

the quantitative data relating to the sum of the amounts a), b) and optionally c), and subsequent shaping to give spherical or lenticular particles having diameters of 0.5 to 4 mm or by passing the extruded strip between two counterrotating rolls having hollows lying opposite one another in the roll section, the shape of the hollows determining the tablet shape.

**2.** A drug form as claimed in claim 1, comprising maltodextrins as components b).

**3.** A drug form as claimed in claim 1 or 2, having an active substance content of from 0.1 to 90% by weight.

**Revendications**

**1.** Formes galéniques solides, pouvant être obtenues par extrusion d'une masse fondue exempte de solvant, contenant outre une ou plusieurs substances actives un mélange de

a) 10 à 99 % en poids d'un ou plusieurs homo- ou copolymères de N-vinylpyrrolidone hydrosolubles, pouvant être transformés par voie thermoplastique, b) 1 à 90 % en poids d'amidon dégradé, et c) 0 à 50 % en poids d'un ou plusieurs adjuvants pharmaceutiques classiques,

tandis que les indications de quantités se rapportent à la somme des quantités a), b) et éventuellement c), et mise en forme subséquente pour l'obtention de particules en forme de billes ou de lentilles ayant des diamètres de 0,5 à 4 mm ou pour l'introduction du jonc extrudé entre deux rouleaux éventuellement entraînés ayant des évidements en vis-à-vis l'un de l'autre dans l'élément cylindre, tandis que la forme des évidements correspond à la forme des comprimés.

**2.** Formes galéniques selon la revendication 1, contenant comme composant b) de la maltodextrine.

**3.** Formes galéniques selon la revendication 1 ou 2, ayant une proportion de substance active de 0,1 à 90 % en poids.